# EUROPEAN PATENT APPLICATION

(11) **EP 1 475 104 A1**
(43) Date of publication of application: **10.11.2004**
(21) Application number: 04000192.7
(22) Date of filing: 08.01.2004
(51) Int. Cl.: A61K 47/42, A61K 38/18

(54) **Stabilized proteinic preparation**

(30) Priority: 14.01.2003 JP 2003005431; 15.12.2003 JP 2003416440
(71) Applicant: Nipro Corporation, Osaka-shi, Osaka 531-8510 (JP)
(72) Inventor: Ol, Kiyomoto, Osaka-shi Osaka 531-8510 (JP); Sato, Makoto, Osaka-shi Osaka 531-8510 (JP); Umeda, Naoki, Osaka-shi Osaka 531-8510 (JP)
(74) Representative: Keller, Günter, Dr.

(57) **Abstract**

An object of the present invention is to provide a safe proteinic preparation having uniform quality, stability even for preservation for a long period and containing no virus, protein and peptide having pathogenicity and bad affection. [Means for Resolution]

A stabilized proteinic preparation containing (a) a recombinant cell growth factor and (b) one or more member(s) selected from the group consisting of recombinant albumin, recombinant collagen and a treated product of recombinant collagen.

## Description

### BACKGROUND OF THE INVENTION

### 1. FIELD OF THE INVENTION

The present invention relates to a safe and stable proteinic preparation and to utilization of the proteinic preparation.

### 2.DESCRIPTION OF THE PRIOR ART

Proteinic substances used as pharmaceuticals include natural substances derived from human being or derived from animals except human being, recombinant substances produced by transfection of expression vector to microbial cells such as *Escherichia coli* and yeast, mammalian cells, etc. Usually, stabilizers, antioxidants, preservatives, excipients, buffers, isotonic agents, pH adjusting agents, adsorption inhibitors or the like is added to the proteinic substances followed by separately being placed into glassy or plastic containers and put into the market.

When serum albumin is added, that which is derived from animals except human being such as cattle is unable to be used for avoiding antigen-antibody reaction and, accordingly, serum albumin derived from human being (HSA) is inevitable. However, HSA is expensive because it is purified from human blood and is hardly available as well. Moreover, in recent years, there have been a problem of HSA derived from patients suffering from acquired immune deficiency syndrome (AIDS) which is now being spread throughout the world and a problem of infection of hepatitis virus from HSA derived from infected patients. Particularly in Japan, most of blood preparations are relied on import and, therefore, such problems are serious. In addition, HSA contains protease, etc. and, when it is used as a stabilizer for proteinic preparations, there may be an affection by that.

As shown below, serum albumin derived from human being has been used as a stabilizer or the like for preparations. For example, there are an immunoglobulin preparation (Patent Document 1), a modified t-PA preparation (Patent Document 2), a thrombopoietin preparation (Patent Document 3), etc.

Further, when serum albumin derived from human being is used and the substance is derived from human being who is exposed to danger of viral infection or the like, there is a way of thinking that, as a result of such a substance, safety of the preparation to which it is added is worried about and, therefore, a recombinant human serum albumin is used as a stabilizer in live viral vaccine to virus such as varicella zoster, measles, mumps and rubella (Patent Document 4).

Besides the above, collagen, gelatin, etc. are also widely used as stabilizers for proteinic preparations although the used ones are those derived from animals. In addition, a method where a proteinic substance derived from animal is not used as a stabilizer (Patent Document 5) has been developed.

### [Patent Document 1]

Japanese Laid-Open Patent Gazette No. Sho-62-20965

### [Patent Document 2]

Japanese Laid-Open Patent Gazette No. Hei-5-78259

### [Patent Document 3]

Japanese Laid-Open Patent Gazette No. Hei-10-510841

### [Patent Document 4]

Japanese Laid-Open Patent Gazette No. 2001-518447

### {Patent Document 5}

Japanese Patent Specification No. 3,068,033

### SUMMARY OF THE INVENTION

As mentioned above, in the conventional proteinic preparations, components dangerous to human being which are derived from natural animals are used as stabilizers. Stable stabilizers have been demanded for very many proteinic preparations and, moreover, stabilizers which are useful for preservation for a long period have been demanded. Thus, there have been a demand for a safe and stable proteinic preparation containing no virus, protein and peptide which have no pathogenicity for or have no bad affection on a patient to whom the preparation is administered.

In order to solve the above-mentioned problems, the present inventors have carried out studies for stabilized preparations which are safe and in good quality by addition of different recombinant human protein as a stabilizer to a recombinant proteinic preparation of aimed substance in the manufacture of a proteinic preparation. As a result, it has been found that a proteinic preparation in which safety and stability of the proteinic substance are improved is able to be prepared when recombinant human serum albumin, recombinant collagen, a treated product of recombinant collagen or a mixture of two or more of them is added as a stabilizer to a recombinant cell growth factor of an aimed substance whereupon the present invention has been achieved.

Thus, the present invention relates,to:
(1) A stabilized proteinic preparation containing (a) a recombinant cell growth factor and (b) one or more member(s) selected from the group consisting of recombinant albumin, recombinant collagen and a treated product of recombinant collagen;
( 2 ) The proteinic preparation mentioned in the above (1) , wherein the recombinant albumin is a recombinant serum albumin;
(3) The proteinic preparation mentioned in the above (1) , wherein the recombinant albumin is a recombinant human serum albumin;
(4) The proteinic preparation mentioned in the above (1) , wherein concentration of the component (b) is 1 to 300 mg/mL.
(5) The proteinic preparation mentioned in the above (1) , wherein the component (a) is recombinant erythropoietin and the component (b) is a recombinant human serum albumin;
( 6 ) The proteinic preparation mentioned in the above (5) , wherein concentration of the recombinant erythropoietin is 500 to 50,000 IU/mL;
(7) The proteinic preparation mentioned in the above (1) , wherein the component (a) is a recombinant granulocyte colony-stimulating factor and the component (b) is a recombinant human serum albumin;
(8) The proteinic preparation mentioned in the above (7) , wherein concentration of the recombinant granulocye colony-stimulating factor is 25 to 500 µg/mL;
(9) The proteinic preparation mentioned in the above (1) , wherein it is liquid or powdery;
(10) The proteinic preparation mentioned in the above (1) to (9), wherein it is received in a glass, plastic or metallic container;
(11) A process for the production of the proteinic preparation mentioned in the above (1), characterized in that, (a) a recombinant cell growth factor and (b) one or more member(s) selected from the group consisting of recombinant albumin, recombinant collagen and a treated product of recombinant collagen are uniformly mixed;
(12) A use of the proteinic preparation mentioned in the above (1) to (10) as a medicine;
(13) A method for prevention or therapy of diseases associated with cell growth deficiency, characterized in that, the proteinic preparation mentioned in the above (1) to (10) is used.

### DETAILED DESCRIPTION OF THE INVENTION

With regard to the cell growth factor in the recombinant cell growth factor contained in the proteinic preparation of the present invention, there is no particular limitation so far as it is a factor having an ability of promoting the cell growth either directly or indirectly and its examples are hormones such as insulin, glucagon, follicle-stimulating hormone, growth hormone, parathyroid hormone and steroid hormone; B cell growth factor (BCGF); cartilage derived growth factor (CDGF); cell division factor (CDF); colony-stimulating factor (CSF) such as granulocyte colony-stimulating factor (G-CSF), macrophage colony-stimulating factor (M-CSF) and granulocyte-macrophage colony-stimulating factor (GM-CSF); endothelial cell growth factor (ECGF); epidermal growth factor (EGF); erythropoietin (EPO) (α-, β - and ω- type , sugar chain modified type and modified EPO); fibroblast growth factor (FGF); macrophage-derived growth factor (MDGF); nerve growth factor (NGF); platelet-derived growth factor (PDGF); somatomedin A; transferrin; stem cell growth factor (SCF); tumor growth factor (TGF); heptatocyte growth factor (HGF); transforming growth factor (TGF)-α and -β; and thrombopoietin (TPO).

Among the above, it is preferred as a cell growth factor to use a growth factor acting on blood system including colony-stimulating factor (CSF) such as granulocyte colony-stimulating factor (G-CSF), macrophage colony-stimulating factor (M-CSF) and granulocyte-macrophage colony-stimulating factor (GM-CSF); erythropoietin (EPO) (α-, β- and ω-type, sugar chain modified type and modified EPO); stem cell growth factor (SCF); or thrombopoietin (TPO) and it is more preferred to use erythropoietin (EPO) (α-, β- and ω-type, sugar chain modified type and modified EPO).

In the present invention, its characteristic feature is to use a recombinant of the above-mentioned cell growth factors. The recombinant cell growth factor is produced, for example, by transfection of expression vector to microbes such as *Escherichia coli* and yeast, silkworm or mammalian cells, etc. Since a genetic recombination technique has been well established already, a recombinant cell growth factor is able to be easily produced according to a method mentioned in publicly known documents such as "Molecular Cloning" (Sambrook, et al. , 1989). To be more specific, recombinant DNA coding for erythropoietin for example is mentioned in publicly known documents such as Japanese Laid-Open Patent Gazettes No. Hei-2-17156 and No. Hei-6-55144, etc.

The proteinic preparation of the present invention contains one or more member(s) selected from the group consisting of recombinant albumin, recombinant collagen and a treated product of recombinant collagen. Since it/they act(s) as a stabilizer for the recombinant cell growth factor, it/they may be sometimes called "stabilizer" as a general name therefor in the present specification.

There is no particular limitation for the recombinant albumin or the recombinant collagen used in the present invention so far as it is prepared by means of a genetic recombination technique and is well purified to an extent of being applicable as a medicine and publicly known ones may be utilized.

There is no limitation for the origin of the recombinant albumin used in the present invention and it includes, for example, that derived from animal albumin such as egg albumin, serum albumin, milk albumin and muscle albumin (myogen) and that derived from plant albumin such as leucocin, legumelin and ricin. Among them, in the present invention, it is preferred to use a recombinant albumin derived from serum albumin of the same animal as the object to be administered and it is more preferred to use a recombinant albumin derived from human serum albumin.

With regard to the recombinant albumin, there is no particular limitation so far as it is albumin which is produced by a recombinant albumin-producing host prepared by way of a genetic operation. Preferably, however, that which contains substantially no contaminant derived from the host for the production (such as protein and DNA) is used and, more preferably, that which is collected and purified by known separation means and purification means, respectively, from culture filtrate or cell body, cell after incubation of recombinant albumin-producing host by known means is used. It is also possible to utilize transgenic animals or transgenic plants (e.g., Japanese Patent Laid-Open Gazettes No. Hei-9-509565 and No. Hei-10-504289).

With regard to a process for the production of the recombinant albumin, there is specifically the following method. With regard to the host for the preparation of the recombinant albumin used in the present invention, there is no particular limitation so far as it is prepared by way of a genetic operation and, besides that which has been mentioned in the publicly known documents already, even that which will be developed in future may be appropriately utilized. To be more specific, its examples are microbes (such as *Escherichia coli,* yeast and *Bacillus subtilis*), animal cells, etc. which are made into a recombinant albumin-producing ones by way of a genetic operation. Particularly, yeast or, preferably, that of genus *Saccharomyces* (such as *Saccharomyces cerevisiae*) or genus *Pichia* (such as *Pichia pastoris*) is used. Auxotrophic strain or antibiotic-sensitive strain may be used as well. More preferably, *Saccharomyces cerevisiae* AH 22 strain (a, his 4, leu 2, can 1) and *Pichia pastoris* GTS 115 strain (his 4) are used.

A method for the preparation of the recombinant albumin-producing host as such, a method for the production of the recombinant albumin by incubation of the host and a method for the separation and collection of the recombinant albumin from the cultured substance may be carried out by adopting a publicly known means or a means similar thereto. For example, with regard to a method for the preparation of the recombinant albumin-producing host, its examples are a method using common HSA gene (e.g., Japanese Patent Laid-Open Gazettes No. Sho-58-56684, No. Sho-58-90515 and No. Sho-58-150517), amethod using a novel HSA gene (e. g. , Japanese Patent Laid-Open Gazettes No. Sho-62-29985 and No. Hei-1-98486), a method using a synthetic signal sequence (e.g., Japanese Patent Laid-Open Gazette No. Hei-1-240191), a method using a serum albumin signal sequence (e.g., Japanese Patent Laid-Open Gazette No. Hei-2-167095), a method where recombinant plasmid is integrated on chromosome (e.g., Japanese Patent Laid-Open Gazette No. Hei-3-72889), a method where hosts are fused each other (e.g., Japanese Patent Laid-Open Gazette No. Hei-3-53877), a method where mutation is carried out on a methanol-containing medium, a method using modified AOX2 promoter (e.g., Japanese Patent Laid-Open Gazettes No. Hei-6-90768 and No. Hei-4-299984), expression of HSA by *Bacillus subtilis* (e.g., Japanese Patent Laid-Open Gazette No. Sho-62-25133), expression of recombinant albumin by yeast (e.g. , Japanese Patent Laid-Open Gazettes No. Sho-60-41487, No. Sho-63-39576 and No. Sho-63-74493) and expression of recombinant albumin by *Pichia* yeast (e.g., Japanese Patent Laid-Open Gazette No. Hei-2-104290).

Among the above, a method where mutation is carried out in a methanol-containing medium is concretely conducted as follows. Thus, at first, plasmid having a transcription unit whereby HSA is expressed is introduced into an AOX1 gene region of GTS 115 strain (Deposition No. Y-15851 at the NRRL) under the control of an AOX1 promoter by a conventional method whereupon a transformant is prepared (refer to the Japanese Patent Laid-Open Gazette No. Hei-2-104290). The transformant has a weak growing ability in a methanolic medium. Therefore, the transformant is incubated in a methanol-containing medium to carry out a mutation and only the strain which is able to grow is recovered. At that time, an example of the methanolic concentration is about 0.0001 to 5%. The medium may be any of artificial and natural media. Examples of the condition for the incubation are at about 15 to 40°C for about 1 to 1,000 hours.

With regard to a method for the incubation of a recombinant albumin-producing host, besides the methods mentioned in the above gazettes, its examples are a method where highly concentrated glucose or methanol or the like is appropriately supplied little by little by means of a fed-batch culture (semi-batch culture) whereupon highly concentrated cells and product are prepared avoiding a high-concentration substrate inhibition (e.g., Japanese Patent Laid-Open Gazette No. Hei-3-83595) and a method where fatty acid is added to a medium whereupon production of the recombinant albumin is enhanced (e.g., Japanese Patent Laid-Open Gazette No. Hei-4-293495).

Various methods have been proposed for a process where the recombinant albumin produced by an incubation treatment is isolated and purified from the components derived from the host cells, incubated component, etc. preventing their contamination or, preferably, without a substantial contamination thereof. For example, as a method which has been conventionally carried out, an example is a method where a yeast culture containing a recombinant albumin is subjected to compression, treatment with ultrafilter membrane, heating treatment and treatment with ultrafilter membrane and then subjected to the steps such as treatment with cation-exchanger, hydrophobic chromatographic treatment, treatment with anion-exchanger, etc. (e.g., Japanese Patent Laid-Open Gazette No. Hei-5-317079; *Biotechnology of Blood Proteins,* volume 227, pages 293 to 298, published in 1993). There are also other reports that, after the above-mentioned conventional method, the product is further subjected to a step of treatment with chelate resin or treatment with boric acid and its salt (e.g., Japanese Patent Laid-Open Gazettes No. Hei-6-56883 and No. Hei-6-245789). It is also possible to use a streamline method where, after subjecting the yeast culture to a heating treatment, an adsorption fluidized bed technique is used (e.g., Japanese Patent Laid-Open Gazette No. Hei-8-116985). The recombinant albumin which is prepared and purified as such may, for example, be subjected to a treatment such as sterilizing heating, treatment with ultrafilter membrane, addition of stabilizer, sterile filtration, dispensing and freeze-drying.

The recombinant collagen used in the present invention may also be easily produced by a method mentioned in known documents such as Japanese Patent Laid-Open Gazette No. Hei-8-23979. The treated product of the recombinant collagen used in the present invention may be that which is prepared by subjecting a recombinant collagen to a certain treatment and its examples are a hydrolyzed product of recombinant collagen, a product where a substituent (such as fatty acid or sugar chain) is bonded to side chain, etc.

The proteinic preparation according to the present invention may be produced by a uniform mixing of (a) a recombinant cell growth factor with (b) one or more member(s) selected from the group consisting of recombinant albumin, recombinant collagen and a treated product of recombinant collagen together, if desired, with (c) other additive within such an extent that it does not deteriorate the object of the present invention.

Examples of the additive used in the present invention are saccharides such as dextran, mannitol, sorbitol, inositol, glucose, fructose, lactose, xylose, mannose, maltose, sucrose and raffinose; inorganic salts such as sodium chloride, calcium chloride, sodium phosphate, potassium phosphate and sodium hydrogen carbonate; organic salts such as citrate and acetate; sulfur-containing reducing agents such as glutathione, thioctic acid, sodium thioglycolate, thioglycerol, α-monothioglycerol and sodium thiosulfate; sugar alcohols such as polyethylene glycol and dextran; surfactants such as polyoxyethylene sorbitan alkyl ester; amino acids such as leucine, phenylalanine, sodium glutamate, arginine hydrochloride, histidine hydrochloride and lysine hydrochloride; chelating agents such as EDTA disodium; lecithin; copolymers of ethylene oxide with propylene oxide; hydroxypropyl cellulose; methyl cellulose; polyoxyethylene hydrogenated castor oil; polyethylene glycol; ethylenediamine; trometamol; and maleic anhydride. Preferable examples of the additive are ethylenediamine, trometamol and maleic anhydride. In the present invention, one or more additive(s) selected from the above group can be used. Depending upon physical property or use of the recombinant cell growth factor used for the proteinic preparation according to the present invention, the combination varies and persons skilled in the art are able to easily select the combination.

Amount of the recombinant cell growth factor contained in the proteinic preparation of the present invention may be decided depending upon type of the recombinant cell growth factor used, type of the diseases to be treated, age of the patient, etc. For example, in the case of a recombinant erythropoietin preparation, amount of the recombinant erythropoietin in the preparation is about 250 to 100,000 IU/container, preferably, about 500 to 50,000 IU/container while, in the case of a recombinant G-CSF preparation, amount of the recombinant G-CSF in the preparation is about 10 to 1,000 µg/container, preferably, about 25 to 500 µg/container where volume of one preparation is about 0.5 to 5.0 mL/container, preferably about 0.5 to 2 mL/container. Amount of one or more member(s) selected from the group consisting of recombinant albumin, recombinant collagen and a treated product of recombinant collagen which is/are a stabilizer contained in the proteinic preparation of the present invention is about 1 to 300 mg/container, preferably about 1 to 100 mg/container or, more preferably, about 1 to 50 mg/container. Volume of one preparation is about 0.5 to 5 mL/container, preferably, about 0.5 to 2 mL/container.

The proteinic preparation of the present invention may be in any dosage form such as liquid, solid and semi-solid and may be used, for example, as tablets, pills, capsules, diluted powder, granules, suppositories, injections, pastes, ointments, creams, gels, gel-like creams, lotions, emulsions, suspensions, poultices, plasters, liniments, aerosols, syrups, oral agents, eye drops or nose drops. The tablets may be coated ones such as sugar-coated tablets, gelatin-enclosed tablets, enteric-coated tablets or film-coated tablets or may be double-layered tablets or multi-layered tablets. Such pharmaceutical preparations are able to be manufactured by a known or common method *per se* in the field of pharmaceutical preparations.

It is preferred that the proteinic preparation of the present invention has the following dosage form. Thus, it includes (a) a preparation where a recombinant cell growth factor and a stabilizer are made to coexist in a solution; (b) a preparation where a recombinant cell growth factor and a stabilizer each is in a separate solution and both are combined in such a manner that they are mixed upon use; (c) a preparation where freeze-dried powder containing a recombinant cell growth factor and a stabilizer is combined with a solution for dissolving by which the above freeze-dried powder is dissolved upon use; (d) a preparation where there are combined freeze-dried powder containing a recombinant cell growth factor, freeze-dried powder containing a stabilizer and solution for dissolving by which the above two kinds of freeze-dried powders are dissolved upon use; (e) a preparation where freeze-dried powder containing a recombinant cell growth factor is combined with a solution containing a stabilizer and, upon use, the above freeze-dried powder is dissolved in the solution containing a stabilizer; etc.

In the proteinic preparation of the present invention, an additive for the production of preparation may be compounded during the course of production of the preparation.

For example, when the proteinic preparation according to the present invention is in a dosage form of a parenteral liquid preparation such as injection, examples of the additive for the preparation are solubilizers, suspending agents, isotonic agents, buffers and soothing agents. Examples of the solubilizing agent are polyethylene glycol, polypropylene glycol, D-mannitol, benzyl benzoate, ethanol, trisaminomethane, cholesterol, triethanolamine, sodium carbonate and sodium citrate. Examples of the suspending agent are surfactants such as stearyl triethanolamine, sodium laurylsulfate, laurylaminopropionic acid, lecithin, benzalkonium chloride, benzethonium chloride and glycerol monostearate; and hydrophilic polymers such as polyvinyl alcohol, polyvinylpyrrolidone, carboxymethyl cellulose sodium, methyl cellulose, hydroxymethyl cellulose, hydroxyethyl cellulose and hydroxypropyl cellulose. Examples of the isotonic agent are sodium chloride, glycerol and D-mannitol. The buffer includes a buffer solution of phosphate, acetate, carbonate, citrate, etc. Examples of the soothing agent are benzyl alcohol, etc.

Examples of the solvent for a parenteral liquid preparation are alcohol and water for injection. Preparations for administering into blood vessel such as injection and drip among the parenteral liquid preparation may be preferably prepared using an aqueous medium which is isotonic to body fluid. For example, in the case of injection preparation, an aqueous medium selected from salt solution, glucose solution and a mixture of salt solution and glucose solution is used and the preparation can be prepared as solution, suspension or dispersion together with an appropriate adjuvant according to the conventional method.

When the proteinic preparation according to the present invention is a solid preparation such as freeze-dried powder, examples of the additive for the preparation are excipients such as lactose, glucose, sucrose and mannitol; disintegrators such as starch and sodium alginate; lubricants such as magnesium stearate and talc; binders such as polyvinyl alcohol and hydroxypropyl cellulose; surfactants such as fatty acid esters; plasticizers such as glycerol; etc.

The proteinic preparation according to the present invention may be made into the final product by the publicly known method. For example, when the proteinic preparation according to the present invention is a liquid preparation, the proteinic preparation according to the present invention is received in a glassy, plastic or metallic container by a known method *per se* and the receipt is carried out under an aseptic condition or sterilization is conducted after the receipt whereupon the final product is manufactured.

The proteinic preparation according to the present invention may be used for various uses depending upon type, amount, etc. of the recombinant cell growth factor contained therein. For example, the proteinic preparation according to the present invention may be used for prevention or therapy of the diseases associated with cell growth deficiency. To be more specific, erythropoietin is a sugar protein of molecular weight of about 35,000 comprising 166 amino acids and having a function of maturation and differentiation to blood cells and, when erythropoietin is used for an anemic state such as a patient treated with dialysis, it has a function of increasing the blood cells without a blood transfusion. Therefore, the proteinic preparation according to the present invention containing a recombinant erythropoietin as a cell growth factor is able to be used for prevention and/or therapy of anemia.

CSF acts on precursor cells for various blood cells in bone marrow, promotes differentiation induction to and growth of granulocytes, macrophages, etc., promotes flow of the produced blood cells to peripheral blood and has an action of enhancing the function of differentiated and induced various blood cells. Therefore, the proteinic preparation according to the present invention containing CSF as a cell growth factor is able to be applied to agranulocytosis as a result of anti-cancer therapy, transplantation of bone marrow, AIDS, etc. To be more specific, the proteinic preparation according to the present invention containing a recombinant M-CSF, the proteinic preparation according to the present invention containing a recombinant G-CSF and the proteinic preparation according to the present invention containing a recombinant GM-CSF are able to be used as preventive or therapeutic agents for patients in gynecologic field and patients transplanted with bone marrow, for agranulocytosis and neutropenia as a result of chemotherapy and radiotherapy in patients suffering from lung cancer and malignant lymphoma and for patients suffering from AIDS, aplastic anemia, MDS (myelodysplastic syndrome), etc., respectively.

### WORKING EXAMPLES

In order to further illustrate the present invention, the following Examples are submitted although it goes without saying that the present invention is not limited thereto.

### Example 1

| | |
|---|---|
| Recombinant human erythropoietin | 1500 IU |
| Recombinant human serum albumin | 4.0 mg |
| Sodium hydrogen phosphate | 8.77 mg |
| Potassium dihydrogen phosphate | 0.75 mg |
| Sodium chloride | 17.5 mg |
| Water for injection | q. s. |

A solution was prepared according to the above composition amounts to make the total volume 2 mL, aseptically dispensed in vials, ampoules or syringes and tightly sealed to prepare a solution preparation. In the case of vials or ampoules, a tightly closed freeze-dried preparation was also prepared by conducting freeze-drying by a conventional method after dispensing.

### Example 2

| | |
|---|---|
| Recombinant human erythropoietin | 1500 IU |
| Recombinant human serum albumin | 5.0 mg |
| Sodium hydrogen phosphate | 8.0 mg |
| Potassium dihydrogen phosphate | 1.0 mg |
| Sodium chloride | 18.0 mg |
| Water for injection | q. s. |

A solution was prepared according to the above composition amounts to make the total volume 2 mL, aseptically dispensed in syringes and tightly sealed to prepare a solution preparation. The solution preparation did not show coloration of the solution even after stored for three months at room temperature and rarely showed changes in the content but was stable.

### Example 3

| | |
|---|---|
| Recombinant human erythropoietin | 1500 IU |
| Recombinant human serum albumin | 2.0 mg |
| Sodium hydrogen phosphate | 8.77 mg |
| Potassium dihydrogen phosphate | 0.75 mg |
| Polysorbate 80 | 0.1 mg |
| Sodium chloride | 17.5 mg |
| Distilled water for injection | q. s. |

A solution was prepared according to the above composition amounts to make the total volume 2 mL, aseptically dispensed in syringes and tightly sealed to prepare a solution preparation. The solution preparation rarely showed changes in appearance and titer even after stored for three months at room temperature but was stable.

### Example 4

| | |
|---|---|
| Recombinant human erythropoietin | 3000 IU |
| Recombinant human serum albumin | 2.0 mg |
| Ethylenediamine | 2.0 mg |
| Distilled water for injection | q. s. |

A solution was prepared in such a manner that the above composition amounts were contained in per 2mL of the solution. Sodium chloride was dissolved in the solution to make the osmotic pressure ratio between 0.9 and 1.1, then the pH was adjusted to 6.0 with citric acid hydrate. A given amount (2 mL) of the resulting solution was aseptically dispensed in prefilled glass syringes and tightly sealed with butyl rubber cap and gasket to prepare a solution preparation.

### Example 5

The solution preparation was prepared in a manner similar to that in Example 4, except that 3.0, 4.0 or 5.0 of recombinant human serum albumin was used.

### Example 6

| | |
|---|---|
| Recombinant human erythropoietin | 3000 IU |
| Recombinant human serum albumin | 2.0 mg |
| Trometamol | 0.4 mg |
| Distilled water for injection | q. s. |

A solution was prepared in such a manner that the above composition amounts were contained in per 2mL of the solution. Sodium chloride was dissolved in the solution to make the osmotic pressure ratio between 0.9 and 1.1, then the pH was adjusted to 6.0 with 10 mmol of phosphate buffer. A given amount (2 mL) of the resulting solution was aseptically dispensed in prefilled glass syringes and tightly sealed with butyl rubber cap and gasket to prepare a solution preparation.

### Example 7

The solution preparation was prepared in a manner similar to that in Example 4, except that 3.0, 4.0 or 5.0 of recombinant human serum albumin was used.

### Example 8

| | |
|---|---|
| Recombinant human erythropoietin | 3000 IU |
| Recombinant human serum albumin | 2.0 mg |
| Maleic Anhydride | 10.0 mg |
| Distilled water for injection | q. s. |

A solution was prepared in such a manner that the above composition amounts were contained in per 2mL of the solution. Sodium chloride was dissolved in the solution to make the osmotic pressure ratio between 0.9 and 1.1, then the pH was adjusted to 6.0 with sodium hydroxide. A given amount (2 mL) of the resulting solution was aseptically dispensed in prefilled glass syringes and tightly sealed with butyl rubber cap and gasket to prepare a solution preparation.

### Example 9

The solution preparation was prepared in a manner similar to that in Example 4, except that 3.0, 4.0 or 5.0 of recombinant human serum albumin was used.

### INDUSTRIAL APPLICABILITY

The proteinic preparation of the present invention uses recombinants for both cell growth factor contained therein and stabilizer therefor and, accordingly, it is a safe and stable proteinic preparation containing no virus, protein and peptide having pathogenicity and bad affection.

## Claims

1. A stabilized proteinic preparation containing (a) a recombinant cell growth factor and (b) one or more member(s) selected from the group consisting of recombinant albumin, recombinant collagen and a treated product of recombinant collagen.

2. The proteinic preparation according to claim 1, wherein the recombinant albumin is a recombinant serum albumin.

3. The proteinic preparation according to claim 1, wherein the recombinant albumin is a recombinant human serum albumin.

4. The proteinic preparation according to claim 1, wherein concentration of the component (b) is 1 to 300 mg/mL.

5. The proteinic preparation according to claim 1, wherein the component (a) is recombinant erythropoietin and the component (b) is a recombinant human serum albumin.

6. The proteinic preparation according to claim 5, wherein concentration of the recombinant erythropoietin is 500 to 50,000 IU/mL.

7. The proteinic preparation according to claim 1, wherein the component (a) is a recombinant granulocye colony-stimulating factor and the component (b) is a recombinant human serum albumin.

8. The proteinic preparation according to claim 7, wherein concentration of the recombinant granulocyte colony-stimulating factor is 25 to 500 µg/mL.

9. The proteinic preparation according to claim 1, wherein it is liquid or powdery.

10. The proteinic preparation according to claims 1 to 9, wherein it is received in a glass, plastic or metallic container.

11. A process for the production of the proteinic preparation mentioned in claim 1, **characterized in that**, (a) a recombinant cell growth factor and (b) one or more member(s) selected from the group consisting of recombinant albumin, recombinant collagen and a treated product of recombinant collagen are uniformly mixed.

12. Use of a protein preparation according to claims 1-10 for the preparation of a medicament.

13. Use according to claim 12, wherein the medicament is intended for prevention or therapy of diseases associated with cell growth deficiency.
